# EUROPEAN PATENT APPLICATION

(11) **EP 4 148 130 A1**
(43) Date of publication of application: **15.03.2023**
(21) Application number: 21461586.6
(22) Date of filing: 09.09.2021
(51) Int. Cl.: C12N 15/11, B82Y 5/00

(54) **TOPOGAMI AND METHOD FOR MAKING INTERLOCKED SINGLE STRANDED DNA RINGS**

(71) Applicant: UNIWERSYTET JAGIELLONSKI, 31-007 Kraków (PL)
(72) Inventor: Heddle, Jonathan, 30-394 Kraków (PL); Sakai, Yusuke, Kawanishi, 1-7-17 (JP); Wilkens, Gerrit, 30-513 Kraków (PL)
(74) Representative: Witek, Rafal

(57) **Abstract**

It has been disclosed a method for the construction of single stranded DNA catenanes at the kilobase length scale, which can be used for the assembling scaffolds of covalently connected DNA origamis.

## Description

### Technical Field

The present invention relates to a method for the construction of single stranded DNA catenanes at the kilobase length scale. The catenanes can be used for the assembling scaffolds of covalently connected DNA origamis.

### Background Art

Topologically linked molecules such as catenanes and rotaxanes are challenging and fascinating targets in supramolecular chemistry [1]. They achieved particular prominence after Sauvage's demonstration of the templated approach to molecular catenane synthesis in 1983 [2, 3] with Stoddart demonstrating a rotaxane almost a decade later [4]. Since then there have been many examples of topological molecules [1] and demonstrated applications include as nanometric electronic switches, liquid crystals and other new materials [5].

DNA origami is a DNA nanotechnology in which a long single-stranded DNA "scaffold" is shaped by the action of many short "staple" strands which bind to cognate sequences distributed throughout the scaffold [6, 7]. Typically, the scaffold strand is thousands of bases long, providing enough material for the construction of complex and rigid structures such as dynamic containers whose opening can be programmed in response to stimuli [8, 9]. While customization of scaffolds can be challenging, numerous examples have been reported including those that are shortened [10, 11], extended [12-14] or otherwise modified to provide arbitrary length and sequences [15-18].

DNA origami is a widely used DNA nanotechnology allowing construction of 2D and 3D nanometric shapes. The designability and rigidity of DNA origami makes it an ideal material for construction of topologically linked molecules such as catenanes which are attractive for their potential as motors and molecular machines (eg. US2013224859, WO15177666, US2017108517, CN106770049, CN107083389, CN107124178, CN107469088, WO18129333, US2020031663, KR102129784).

Efforts to connect together discrete DNA origami structures have included hybridisation of sticky ends [19] or base stacking [7, 20, 21, 22] and have achieved gigadalton scale structures [23] and fully addressable semi-micrometre scale tiles [24].

Topologically linked DNA molecules are known to occur in nature [25] and are also attractive as artificial constructs in DNA nanotechnology. In recent years catenated ssDNA rings have been designed and produced in vitro using a number of methods. Typically, these involve enzymatic ligation of short linear DNA components following geometric pre-arrangement by hybridisation of short complementary sequences [26-29] or conjugation with dsDNA-binding moieties [30]. The resulting structures have been shown capable of functioning as switches [26, 31] and rotary motors [27, 32]. However, this approach results in small (sub-200 nt) catenanes, essentially an order of magnitude smaller than required to make complex DNA origami structures. Production of considerably longer catenated ssDNA circles of a length suitable for DNA origami has been demonstrated in work which used Ena/Vasp-like protein and Topoisomerase I [33]. However, this approach showed poor efficiency, generated a range of catenated products, and has not been applied for DNA origami production.

Despite these challenges, topologically linked DNA origami remains an attractive goal due to its high molecular weight and resulting greater structural robustness compared to classical topologically linked molecules. As a result, it has the potential to construct molecular machines with increased functionality and sophistication due to the ability to irreversibly link together discrete DNA origami structures.

Very few examples of topologically linked DNA origamis have been demonstrated to date. The first that we are aware of was in 2010 when a 2-ring DNA origami catenane was produced [34]. This was achieved by splitting a DNA origami mobius strip followed by removal of selected staple strands. In this case, the topology of the two connected scaffold strands is such that decatenation can be achieved without covalent bond breaking. More recently a second 2-ring DNA origami catenane was demonstrated. Here, each of the two rings was made from four individual DNA origamis which were joined together via additional staple strands with the help of a gold particle templated approach [35]. As with the first example, removal of noncovalently bound staple strands results in decatenation of the structure. In contrast a truly catenated scaffold can only be decatenated by covalent bond breaking and may be preferable for the extra stability it would provide to the linked DNA origami components.

Two-component DNA origami structures which could benefit from being produced as true catenanes include rotaxanes, the first of which was demonstrated in 2016 [36] in a structure where two discrete DNA origamis constituted the axle and ring. These were assembled first with the ring in an open form which was then partially wrapped around the axle before being mechanically "clamped" in place by complementary sequence extended from staple strands. More recently another rotaxane was produced from a single template DNA origami wherein the ring and the axle of the rotaxane precursor was connected via ssDNA regions which were then specifically removed by Cas12a to produce the final rotaxane [37].

A common pattern in all topologically linked DNA origami structures to date is that they are specifically constructed such that predetermined DNA origami subunits are positioned with respect to each other. This is followed by disconnection of the "linkers" - a subset of staples, base-pairing at specific sites as well as a part of the scaffold, resulting in a topologically linked product. A consequence of this approach is that the construction method is design specific. The final, folded DNA origami structures are required to be produced first, prior to the topological linkage being formed. This is in contrast to the original DNA origami concept whereby starting with a scaffold strand, almost any arbitrary structure can be designed and produced.

US2014141211 discloses methods for self-assembly of arbitrarily-shaped metal nanostructures using specifically-designed patterns on nucleic acid scaffolds.

US2017066796 discloses compositions and methods for engineering wireframe architectures and scaffolds of increasing complexity by creating gridiron-like DNA structures. A series of four-arm junctions are used as vertices within a network of double-helical DNA fragments. Deliberate distortion of the junctions from their most relaxed conformations ensures that a scaffold strand can traverse through individual vertices in multiple directions. DNA gridirons, ranging from two-dimensional arrays with reconfigurability to multilayer and three-dimensional structures and curved objects, can be assembled.

WO18218646 discloses a method for stepwise assembling multiple nucleic acid origami units into a nucleic acid nanostructure.

However, an alternative, universal method for production of topologically linked DNA origami is still needed.

### Detailed Description of the Invention

### Technical Problem

The present invention is conceived to solve the aforementioned problem, and an object of the present invention is to provide a method of producing a topologically linked two-component DNA origami where the linkage is made at the level of two single-stranded scaffold strands.

### Technical Solution

The solution to the above-described technical problem is provided by subject invention which has been defined by enclosed claims.

### Advantageous Effects

According to subject invention the catenated single stranded DNA circles can be produced and used as a universal scaffold for the production of topologically linked (catenated) DNA origamis where the individual linked structures can be of any arbitrary design. Assembly of these topologically linked DNA origami structures is achieved via a simple one-pot annealing protocol.

Subject invention provides a method for preparation of a pair of topologically interlocked circular ssDNA scaffold rings using Tn3 resolvase. Catenating at this fundamental level has two noteworthy outcomes shown here for the first time. Firstly, the system is universal: as catenation does not depend on the formation of a specific DNA origami structure, the catenated ssDNA rings we produced can, in principle, be used to construct almost any two origami structures in a catenated form. Secondly, unlike topologically linked DNA origami structures to date, the topological linkage can only be undone by breaking a covalent bond (i.e. DNA backbone cleavage) resulting in high stability of the produced structures.

### Brief Description of Drawings

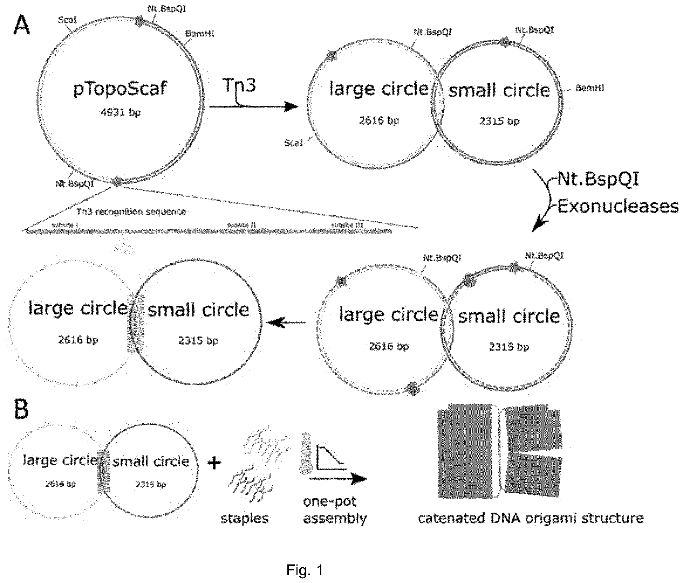

Fig. 1 is an overview of the catenane scaffold production process by a recombination of DNA recombination nicking and exonuclease digestion. (A) First, supercoiled plasmid with two parallel recombination sites are treated with Tn3 resolvase leading to the production of catenane dsDNA circles. Next, both circles are nicked at one specific site on each circle on opposite strands of the original plasmid. One strand of each double stranded circle is removed by exonuclease treatment, generating a single stranded catenane with a 249 nt complementary region between both circles. (B) Single stranded catenanes can be used directly as a scaffold source for one-pot assembly of covalently connected DNA origami structures linked by a scaffold loop.

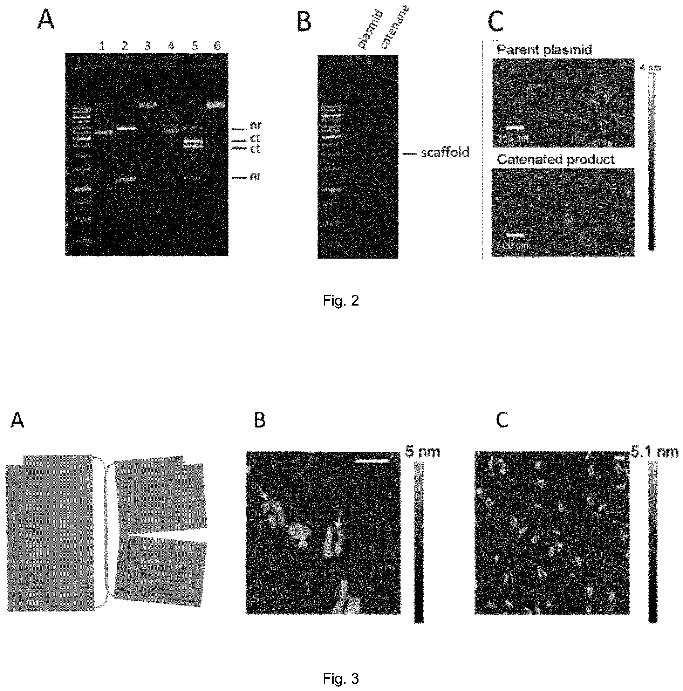

Fig. 2 presents preparation of catenated scaffold from parent plasmid. (A) Catenation of circular dsDNA using Tn3 recombinase. Agarose gel electrophoresis image of (1) undigested parent plasmid (2) plasmid digested with BamHI and ScaI, (3) plasmid nicked with Nt.BspQI, (4) undigested catenane after Tn3 catenation, (5) catenane digested with BamHI and ScaI showing appearance of digestion products from catenane rings (2.3 and 2.6 kbp products), (6) catenane nicked with Nt.BspQI. (B) Exonuclease digestion of nicked plasmid and catenane. Exonuclease treated plasmid appears to be degraded indicating efficient removal of non-catenane plasmid from catenane sample after exonuclease digest. (C) AFM micrograph of plasmid substrate (top, pMA21) and its catenane (bottom) after treatment with Nt.BspQI and Nb.BsmI nickase to remove negative supercoiling. Note that the sequences of those DNA strands differed in one nucleotide from pTopoScaf and its catenane product shown in agarose image. Ladder: GeneRuler 1 kb (Thermo Fisher Scientific).
Fig. 3 shows topogami structure assembly from catenated scaffold. (A) Schematic shape of designed topogami, two different sized rectangles interlocked by a dsDNA loop. (B) Typical AFM image of assembled Topogami structure in 500 nm square. White arrows indicate 249 bp dsDNA loops. Scale bars are 100 nm. (C) Typical AFM image of assembled Topogami structure in 500 nm square. Note that the catenated origami structures are free to rotate relative to each other meaning that the smaller structure may open towards or away from the larger structure. Scale bars are 100 nm.

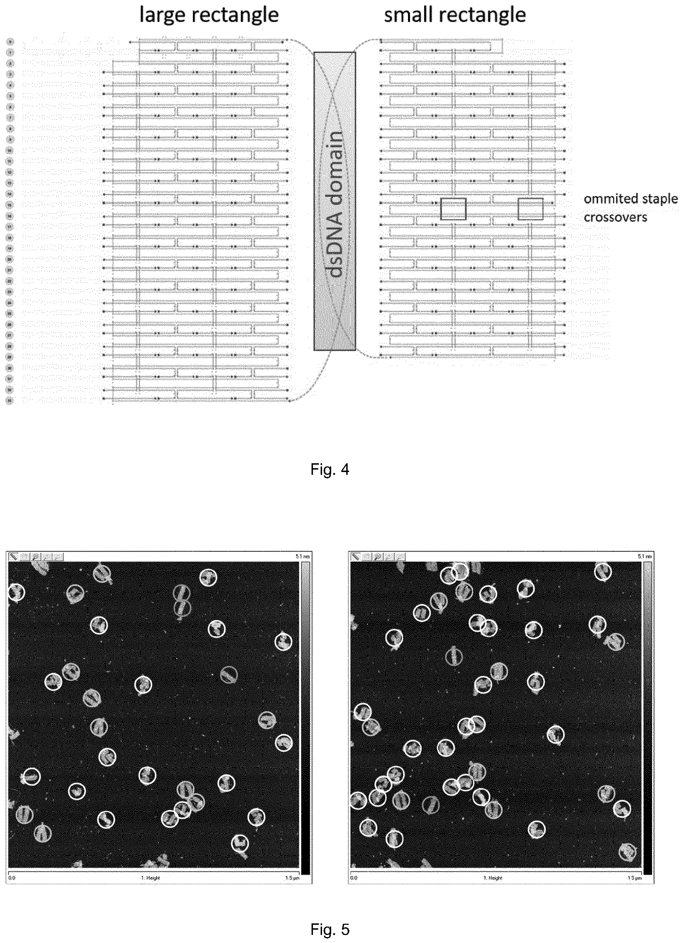

Fig.4 presents cadnano diagram of Rectangle catenanes. Blue and red lines indicated scaffold and staple strands respectively. The "open" appearance of the small rectangle was achieved by removing staple crossovers between the 15th and 16th helices (removed crossovers indicated by red rectangles). The scaffold of both structures is interlocked by an outside scaffold loop shown by the orange dotted lines that forms a double helix domain in between both structures.
Fig. 5 presents AFM image of 184 topogami particles. White: Unclear or broken structures; Orange: Obviously paired rectangles; Blue: Isolated rectangles

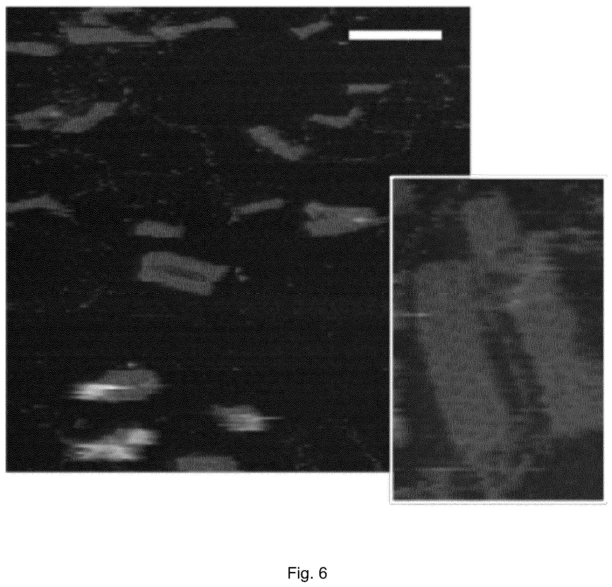

Fig. 6 presents high speed AFM image of topogami. HS-AFM imaging enabled fine and high-resolution observation of topogami structure. Along with horizontal staple crossover pattern on rectangular bodies, double stranded scaffold loop sticking out from corners of each body was observed. Scale bar is 100 nm. Right inset is 128 nm×84 nm square magnified image.

The present invention is further are illustrated by the following Examples, which in no way should be construed as further limiting.

### Examples

### Production of catenated ssDNA scaffold strands

It has been designed pTopoScaf, a vector consisting of two scaffold domains connected by parallel recognition sequences (res) in a negatively supercoiled parent plasmid [38] which are processed by Tn3 resolvase, a serine recombinase [39-41] as outlined in Fig. 1. The enzyme catalyses a site-specific recombination of negatively-supercoiled circular DNA at the res sites to form a supercoiled catenane [42] while the reverse reaction occurs in case of relaxed substrate [38]. The recognition sequence consists of three domains (sites I-III): site I is a cleavage site and sites II-III are required for recombination [39] (Fig. 1).

Nt.BspQI nickase sites have been integrated on opposite strands of the parent vector such that after Tn3 mediated recombination, each ring of the two-ring catenane would bear a single nickase site. This allows conversion of catenated product into ssDNA Nt.BspQI nicking and subsequent exonuclease digestion, while nicked non-catenated parent plasmid will be removed during by exonuclease digestion (Fig. 2B). Due to complementary sequences at the res sites of the two catenated strands, they hybridise to each other forming a 249 bp dsDNA domain, including linker sequence, though this does not change the topology of the catenated product.

Production of catenated scaffolds was confirmed by agarose gel electrophoresis where ScaI/BamHI double digest of the Tn3 treated plasmid indicated the recombination event leading to formation of two smaller circles, each bearing one cutting site (for relative position of BamHI and ScaI cutting sites in the plasmid and catenane please refer to Fig. 1A and Fig. 2). Undigested plasmid and catenane were visible as a major band running at the same height, confirming that the catenane circles were indeed covalently connected (Fig. 2A). This was supported by the consistent pattern of BamHI/Scal double-digest products of each (Fig. 2A).

Further confirmation was provided by AFM analysis which showed clear evidence of catenated product compared to unreacted controls (Fig. 2C). Catenated samples were subsequently treated with Nt.BspQI leading to one major band consisting of catenanes in which each circle in the catenane was nicked once, while the unreacted parent plasmid contained two nicks, one in each strand. As expected, the nicked catenane sample appeared as a single major band on the gel, running higher than the catenane and plasmid samples. Next, plasmid was digested using Exonuclease III and I, removing the parent plasmid while leaving one strand of each of the catenane circles intact (Fig. 2B).

### Production of catenated DNA origami

Using the catenated scaffold, a topologically interlocked DNA origami complex, named "topogami", has been obtained. Topogami consists of two discrete DNA origami structures which are catenated at the level of the scaffold strands. To demonstrate this, two rectangular single layer DNA origamis as the two linked structures (Fig. 3A) wherein the smaller rectangle was partially opened in its central region by omission of staple strands have been chosen, making this structure easier to distinguish in imaging. The formation of the topogami structure was achieved by a typical DNA origami annealing reaction and then analysed by AFM. This showed the presence of an as-designed dimeric structure consisting of two interconnected rectangles with dimensions of 90 nm x 24 nm rectangle and a 80 x 24 nm respectively. By definition the two rings of the topogami must be interlocked. This could prove disruptive if the point of interlocking interferes with the folding of the DNA origami. In addition, described embodiment of topogami design contains 249 complementary bases on each of the two ssDNA rings which could also have disruptive effects if they competed with staple strand binding. Both of these challenges were overcome in a single solution where the complementary sequence was kept discrete from the folded DNA origami. To achieve this, a double helix domain utilising the existing 249 nt complementary sequence was designed to be located between the two connected DNA origamis and was successfully observed in AFM and high-speed AFM imaging (Fig. 3B and Fig. 6). Statistical analysis of AFM image revealed that the majority (69%±2%) of the assembled products were catenated while clearly isolated (decatenated) "half" structures were also observed with substantially lower frequency (31 %±9%). Note that only clear paired rectangles and obviously isolated rectangles were counted to minimize bias (total 96 particles) and the else were separately classified as unclear particles and excluded from above calculation(Fig. 5). Interestingly, in some correctly catenated structures the small rectangle did not appear in the open form possibly due to restrictive effects exerted by the linked large rectangle.

Overall, the data clearly showed the successful demonstration of topologically linked DNA origami used to produce two discrete and covalently catenated DNA origami structures.

### Material and methods

### Scaffold sequences

Large circle sequence (Seq. Id. No. 1) 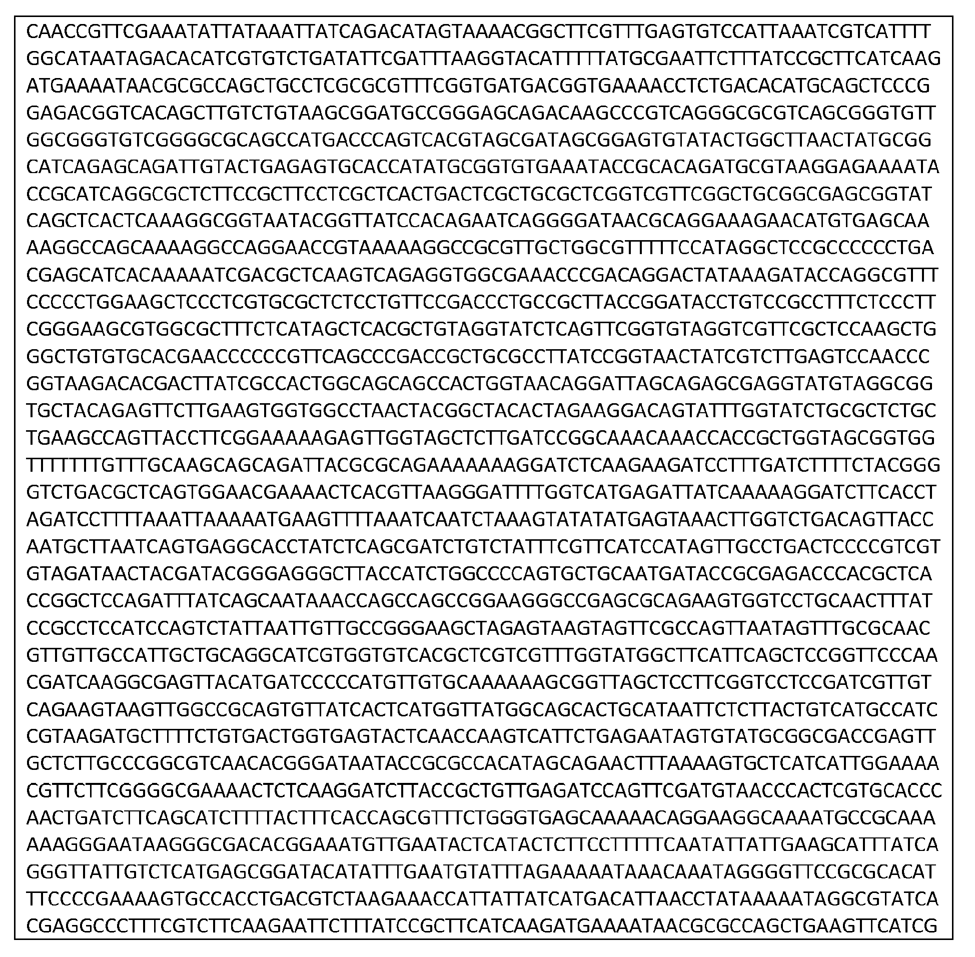 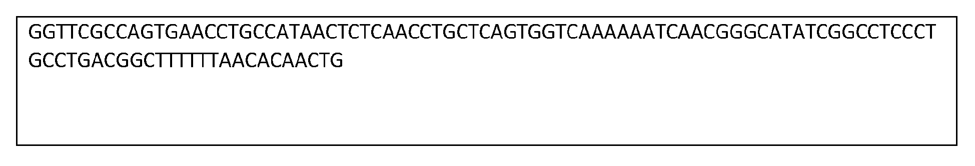
Small circle sequence (Seq. Id. No. 2)

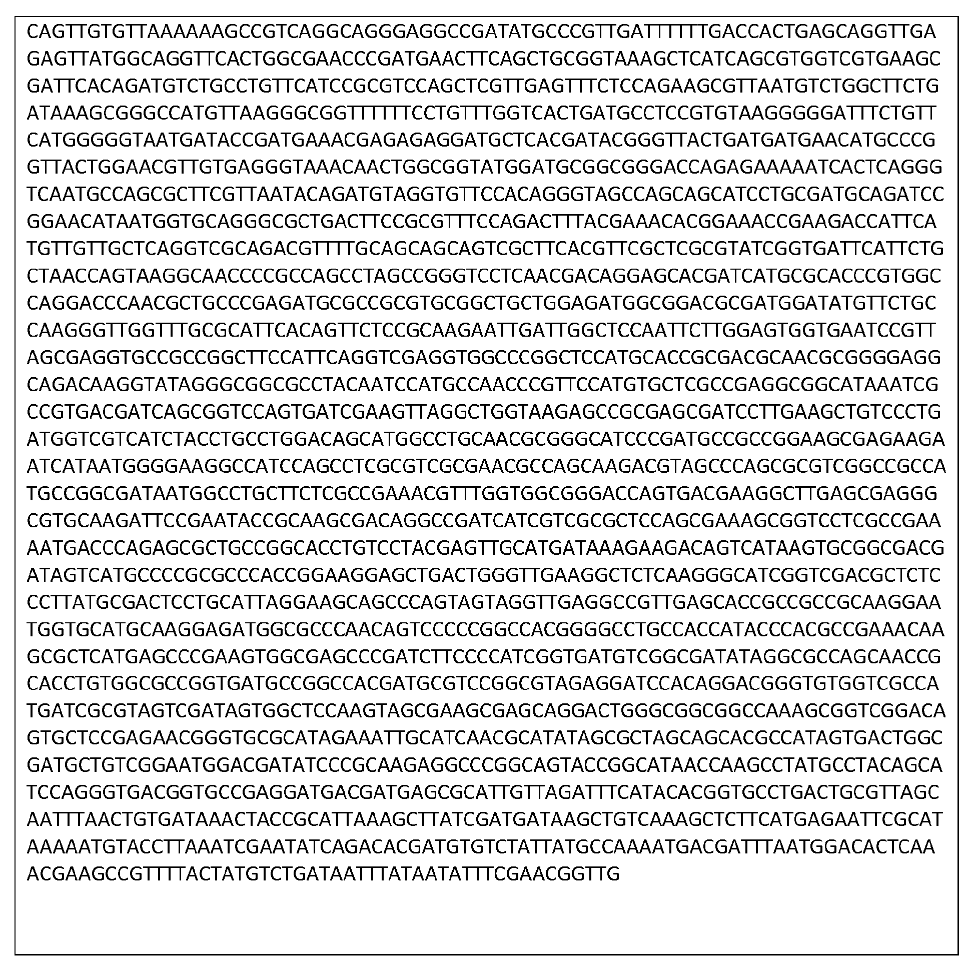

### Staple list for large circle

**Seq. Id. No. 3-51:**

| | |
|---|---|
| 001 0H108-1H110 Large | TATCCGCTTCATCGTAAGCGGATGTTTT |
| 002 1H56-0H39 Large | TCTGACACGTTTCGGTGATGACGGTTTT |
| 003 1H72-3H71 Large | CCCGGAGACGTCAGCGGGTGTTGGTGGCTTAA |
| 004 1H88-1H71 Large | GCTTGTCTAAGATGAAAATAACGCGCCAGCTGCCTCGCGCATGCAGCT |
| 005 2H110-3H110 Large | TTTTCCGGGAGCAGAACTGAGAGTGCTTTT |
| 006 3H33-4H33 Large | TTTTGTCACGTAGCGTCTTCCGCTTCTTTT |
| 007 3H56-1H55 Large | GTGTATACCGGGTGTCGGGGCGCATGAAAACC |
| 008 3H72-5H71 Large | CTATGCGGGATGCGTAAGGAGAAACGTTCGGC |
| 009 3H88-1H87 Large | CAGATTGTCAAGCCCGTCAGGGCGCGGTCACA |
| 010 4H110-5H110 Large | TTTTACCATATGCGGCAAAGGCGGTATTTT |
| 011 5H33-6H33 Large | TTTTCTCGCTCACTGGCAAAAGGCCATTTT |
| 012 5H56-3H55 Large | CGCTCGGTATACCGCATCAGGCGCATAGCGGA |
| 013 5H72-7H71 Large | TGCGGCGAAACGCAGGAAAGAACACGTTTTTC |
| 014 5H88-3H87 Large | AGCTCACTTGTGAAATACCGCACACATCAGAG |
| 015 6H110-7H110 Large | TTTTATACGGTTATCCATCACAAAAATTTT |
| 017 7H56-5H55 Large | GTTGCTGGTGTGAGCAAAAGGCCAACTCGCTG |
| 019 7H88-5H87 Large | CTGACGAGCACAGAATCAGGGGATGCGGTATC |
| 050 20H110-21H110 Large | TTTTCAGTGCTGCAATTGTTGCCGGGTTTT |
| 051 21H33-22H33 Large | TTTTGGAAGGGCCGAGGCATCGTGGTTTTT |
| 053 21H72-23H71 Large | ATCCGCCTATAGTTTGCGCAACGTATTCAGCT |
| 055 22H110-23H110 Large | TTTTAAGCTAGAGTATTACATGATCCTTTT |
| 056 23H33-24H33 Large | TTTTGTCACGCTCGTGTTGGCCGCAGTTTT |
| 057 23H56-21H55 Large | ATGGCTTCTGTTGCCATTGCTGCAGCGCAGAA |
| 058 23H72-25H71 Large | CCGGTTCCTCCTTCGGTCCTCCGATGCATAAT |
| 059 23H88-21H87 Large | AAGGCGAGAGTAGTTCGCCAGTTACCATCCAG |
| 060 24H110-25H110 Large | TTTTCCCATGTTGTGAGATGCTTTTCTTTT |
| 061 25H33-26H33 Large | TTTTTGTTATCACTCGCTCTTGCCCGTTTT |
| 062 25H56-23H55 Large | GGCAGCACTCGTTGTCAGAAGTAACGTTTGGT |
| 063 25H72-27H71 Large | TCTCTTACATTCTGAGAATAGTGTCATAGCAG |
| 064 25H88-23H87 Large | CATCCGTACAAAAAAGCGGTTAGCCAACGATC |
| 065 26H110-27H110 Large | TTTTTGTGACTGGTGAAAACGTTCTTTTTT |
| 066 27H33-28H33 Large | TTTTGCGTCAACACGCGTGCACCCAATTTT |
| 067 27H56-25H55 Large | CCGCGCCAATGCGGCGACCGAGTTATGGTTAT |
| 068 27H72-29H71 Large | AACTTTAACGCTGTTGAGATCCAGCAGCGTTT |
| 069 27H88-25H87 Large | ATCATTGGAGTACTCAACCAAGTCTGTCATGC |
| 070 28H110-29H110 Large | TTTTCGGGGCGAAAAAAAATGCCGCATTTT |
| 071 29H33-30H33 Large | TTTTCTGATCTTCAGTTATTGAAGCATTTT |
| 072 29H56-27H55 Large | ACTTTCACTTCGATGTAACCCACTGGATAATA |
| 073 29H72-31H71 Large | CTGGGTGATGTTGAATACTCATACGATACATA |
| 074 29H88-27H87 Large | AGGAAGGCCTCTCAAGGATCTTACAAGTGCTC |
| 075 30H110-31H110 Large | TTTTAAAAAGGGAATCAAATAGGGGTTTTT |
| 076 31H33-32H33 Large | TTTTTTTATCAGGGTCATTAACCTATTTTT |
| 077 31H56-29H55 Large | CATGAGCGTCTTCCTTTTTCAATACATCTTTT |
| 078 31H72-33H87 Large | TTTGAATGACCTGACGTCTAAGAACGTCTTCAAGAATTCTTTATCCGC |
| 079 31H88-29H87 Large | AAAATAAAAAGGGCGACACGGAAAGCAAAAAC |
| 080 32H110-33H110 Large | TTTTTCCGCGCACATGATGAAAATAACGCG |
| 081 33H33-33H55 Large | TTTTAAAAATAGGCGTATCACGA |
| 082 33H56-31H55 Large | GGCCCTTTACCATTATTATCATGATATTGTCT |
| 083 33H88-31H87 Large | TTCATCAATTCCCCGAAAAGTGCCTATTTAGA |

### Staple list for small circle

**Seq. Id. No. 52-122:**

| | |
|---|---|
| 084 1H145-0H145 Small | TTTTCGCATTGTTAGAGCTGTCAAACATGA |
| 085 1H168-3H167 Small | CACGGTGCGGTGACGGTGCCGAGGTAGCAGCA |
| 086 1H184-1H167 Small | GTTAGCAAATAAACTACCGCATTAAAGCTTATCGATGATAATTTCATA |
| 087 2H222-3H199 Small | TTTTCCGGCAGTACCGGCATAACCAAGCCTACGGAATGG |
| 088 3H145-2H145 Small | TTTTGCATATAGCGCATGACGATGAGTTTT |
| 089 3H168-5H167 Small | CGCCATAGACGGGTGCGCATAGAAAGGACGGG |
| 090 3H184-1H183 Small | GATGCTGTTGCCTACAGCATCCAGCTGACTGC |
| 091 3H200-5H199 Small | ACGATATCGGCGGCCAAAGCGGTCGATAGTGG |
| 092 4H222-3H222 Small | TTTTCAGGACTGGGCCCGCAAGAGGCTTTT |
| 093 5H145-4H145 Small | TTTTAGAGGATCCACATTGCATCAACTTTT |
| 094 5H168-7H167 Small | TGTGGTCGTGATGCCGGCCACGATCCACGCCG |
| 095 5H184-3H183 Small | GCGTAGTCGGACAGTGCTCCGAGATGACTGGC |
| 096 5H200-7H199 Small | CTCCAAGTATAGGCGCCAGCAACCTGGCGAGC |
| 097 6H222-5H222 Small | TTTTATGTCGGCGATAGCGAAGCGAGTTTT |
| 098 7H145-6H145 Small | TTTTTGCCACCATACGCGTCCGGCGTTTTT |
| 099 7H168-9H167 Small | AAACAAGCGCCCAACAGTCCCCCGACGCTCTC |
| 100 7H184-5H183 Small | GCCCGAAGGCACCTGTGGCGCCGGCCATGATC |
| 101 7H200-9H199 Small | CCGATCTTGCCGCAAGGAATGGTGAGCAGCCC |
| 102 8H222-7H222 Small | TTTTTGAGCACCGCCCCCCATCGGTGTTTT |
| 103 9H145-8H145 Small | TTTTGGCATCGGTCGGCCACGGGGCCTTTT |
| 104 9H168-11H167 Small | CCTTATGCGAGCTGACTGGGTTGAAAAATGAC |
| 105 9H184-7H183 Small | CATTAGGACATGCAAGGAGATGGCGCTCATGA |
| 106 9H200-11H199 Small | AGTAGTAGCGACGATAGTCATGCCTACGAGTT |
| 107 10H222-9H222 Small | TTTTCATAAGTGCGGGTTGAGGCCGTTTTT |
| 108 11H145-10H145 Small | TTTTGGTCCTCGCCGAGGCTCTCAAGTTTT |
| 109 11H168-13H167 Small | CCAGAGCGCGATCATCGTCGCGCTCGGCGATA |
| 110 11H184-9H183 Small | ACCTGTCCCCGCGCCCACCGGAAGGACTCCTG |
| 111 11H200-13H199 Small | GCATGATAGTGCAAGATTCCGAATTTGGTGGC |
| 112 12H222-11H222 Small | TTTTTGAGCGAGGGCAAGAAGACAGTTTTT |
| 113 13H145-12H145 Small | TTTTGGCCGCCATGCCCAGCGAAAGCTTTT |
| 114 13H168-15H167 Small | ATGGCCTGCGCCAGCAAGACGTAGATCTACCT |
| 115 13H184-11H183 Small | CGAAACGTACCGCAAGCGACAGGCCTGCCGGC |
| 116 13H200-15H199 Small | GGGACCAGATGGGGAAGGCCATCCCGGGCATC |
| 117 14H222-13H222 Small | TTTTGAAGAATCATATGACGAAGGCTTTTT |
| 118 15H145-14H145 Small | TTTTTGATGGTCGTCCCCAGCGCGTCTTTT |
| 119 15H168-17H167 Small | GCCTGGACGCCGCGAGCGATCCTTGCGGCGCC |
| 120 15H184-13H183 Small | CTGCAACGAGCCTCGCGTCGCGAACTTCTCGC |
| 122 16H222-15H222 Small | TTTTCGTGACGATCAGCCGGAAGCGATTTT |
| 123 17H145-16H145 Small | TTTTCAAGGTATAGGGAAGCTGTCCCTTTT |
| 124 17H168-19H167 Small | TACAATCCCACCGCGACGCAACGCACAGTTCT |
| 125 17H184-15H183 Small | CCGTTCCAAGTTAGGCTGGTAAGAAGCATGGC |
| 126 17H200-19H199 Small | CCGAGGCGTTCCATTCAGGTCGAGTCTTGGAG |
| 127 18H222-17H222 Small | TTTTGTGCCGCCGGCGCATAAATCGCTTTT |
| 128 19H145-18H145 Small | TTTTTTTGCGCATTCGGGGAGGCAGATTTT |
| 129 19H168-21H167 Small | CCGCAAGACGATGGATATGTTCTGCTAGCCGG |
| 130 19H184-17H183 Small | GCTCCAATGTGGCCCGGCTCCATGATGCCAAC |
| 13119H200-21H199 Small | TGGTGAATGCGCCGCGTGCGGCTGATGCGCAC |
| 132 20H222-19H222 Small | TTTTCTGCCCGAGATCCGTTAGCGAGTTTT |
| 133 21H145-20H145 Small | TTTTACCCCGCCAGCCCAAGGGTTGGTTTT |
| 134 21H168-23H167 Small | GTCCTCAATGATTCATTCTGCTAATTCCAGAC |
| 135 21H184-19H183 Small | GCACGATCCTGGAGATGGCGGACGATTGATTG |
| 136 21H200-23H199 Small | CCGTGGCCCAGCAGTCGCTTCACGCCATTCAT |
| 137 22H222-21H222 Small | TTTTACGTTTTGCAGAGGACCCAACGTTTT |
| 138 23H145-22H145 Small | TTTTGACTTCCGCGTCCAGTAAGGCATTTT |
| 139 23H168-25H167 Small | TTTACGAAATCCGGAACATAATGGATGCGGCG |
| 140 23H184-21H183 Small | ACCGAAGATTCGCTCGCGTATCGGCGACAGGA |
| 141 23H200-25H199 Small | GTTGTTGCCACAGGGTAGCCAGCATCAATGCC |
| 142 24H222-23H222 Small | TTTTTGTAGGTGTTCTCAGGTCGCAGTTTT |
| 143 25H145-24H145 Small | TTTTTGGCGGTATGGTGCAGGGCGCTTTTT |
| 144 25H168-27H167 Small | GGACCAGATACTGGAACGTTGTGAATGCCTCC |
| 145 25H184-23H183 Small | ACTCAGGGGCATCCTGCGATGCAGACACGGAA |
| 146 25H200-27H199 Small | AGCGCTTCGATACGGGTTACTGATGGGGTAAT |
| 147 26H222-25H222 Small | TTTTAGGATGCTCACGTTAATACAGATTTT |
| 148 27H145-26H145 Small | TTTTTTTGGTCACTGGGGTAAACAACTTTT |
| 149 27H168-29H167 Small | GTGTAAGGGGCCATGTTAAGGGCGATCAGCGT |
| 150 27H184-25H183 Small | TGTTCATGGATGAACATGCCCGGTGAAAAATC |
| 151 27H200-29H199 Small | GATACCGAAGAAGCGTTAATGTCTCTGCCTGT |
| 152 28H222-27H222 Small | TTTTTGAGTTTCTCCTGAAACGAGAGTTTT |
| 153 29H145-28H145 Small | TTTTCGGTAAAGCTCGTTTTTTCCTGTTTT |
| 154 29H168-27H183 Small | GGTCGTGAAGCGATTCACAGATGTGGCTTCTGATAAAGCGGGGATTTC |
| 155 29H200-29H222 Small | TCATCCGCGTCCAGCTCGTTTTT |

Plasmid pMA21[45] containing two of parallel Tn3 res sites and *Escherichia coli* DH5 (F⁻ λ⁻ Δ(*lacZYA-argF*)U169 *recA1 endA1 hsdR17* (r_{K}⁻, m_{K}⁺) *phoA supE44 thi*-1 *gyrA96 relA1)* amplification host cell were a gift from Prof. Marshall Stark.

### Plasmid mutagenesis

Plasmid pTopoScaf was derived from pMA21 by introducing a second Nt.BspQI nickase site (GCTCTTCN↓) by PCR using the following mutagenic primers:
Nt.BspQI_for 5'GATAAGCTGTCAAAGCTCTTCATGAGAATTCGC3') and
Nt.BspQI_rev 5'AATTCTCATGAAGAGCTTTGACAGCTTATCATCG3').

Amplification was performed in a 20 µL reaction in 1 x HF buffer (NEB), containing 50 ng template DNA, 1 µM of each primer, 500 µM dNTP mix and 1 µL Phusion polymerase. The PCR cycling program was 98 °C for 60 s followed by 20 cycles at 98 °C for 15 s; 55 °C for 15 s and 72 °C for 140 s and a final elongation step at 72 °C for 4 min. After completing the PCR reaction, methylated template DNA was removed by incubation with 1 µL FastDigest *Dpn*I (Thermo Fisher Scientific) for 15 min at 37 °C. An aliquot of 5 µL was transformed into chemo-competent *E. coli* DH5 and plated on Luria-Bertani plates containing 100 µg/ml ampicillin. The resulting mutant plasmid was verified by DNA sequencing.

### Plasmid mutagenesis

Supercoiled plasmid DNA of pTopoScaf was purified from transformed *E. coli* strainDH5. Bacteria were grown over night in LB containing 100 µg/ml ampicillin. Plasmid was isolated using either the GenElute HP Select Plasmid Gigaprep Kit (Sigma Aldrich) or GeneJET Plasmid Miniprep Kit (Thermo Fisher Scientific). DNA concentration was estimated from absorbance at 260 nm using a Nanodrop ND-1000 spectrophotometer. If required DNA was concentrated using EtOH precipitation.

### Preparation of scaffold catenane

Tn3 resolvase was a kind gift of Prof. Marshall Stark. Tn3 resolvase was diluted and stored in storage buffer (20 mM Tris-HCI (pH 7.5), 1 mM DTT, 0.1 mM EDTA, 1 M NaCl, 50 % v/v glycerol). Plasmid catenation was carried out as previously reported [41] with slight modifications. In brief, Tn3 resolvase was diluted 20-fold into 50 µL reaction buffer (10 mM MgCl₂, 0.1 mM EDTA, 50 mM Tris-HCI, pH 8.2) containing 2.5 µg DNA. Reactions were carried out for 2 h at 37 °C. The Tn3 catenation reaction was stopped by heating for 10 min at 70 °C. After cooling to room temperature, 2 µL Nt.BspQI (NEB, 10,000 U/ml) were added and the reaction was incubated for 1 h at 50 °C followed by 20 min at 80 °C. The nicked DNA was diluted with 50 µL 1 x Exo III buffer (6.6 mM Tris-HCL pH 8, 0.66 mM MgCl₂). 1 µL exonuclease III (Thermo Fisher Scientific, 200 U/ µL) and 4 µL exonuclease I (Thermo Fisher Scientific, 10 U/ µL) was added and incubated for 4 h at 37 °C followed by 10 min at 70 °C. Incubations were carried out in 50 µL aliquots in a thermocycler with heated lid. Reactions were analysed by running 100 ng DNA for on a 1.2 % agarose gel followed by subsequent staining with ethidium bromide and imaging by a UV transilluminator. To purify single stranded DNA from enzymes the products were purified by phenol: chloroform extraction, and concentrated 10 times by EtOH precipitation and resuspension in milli-Q water.

### DNA origami preparation

For design of DNA origami structures cadnano was used [46]

DNA origami structures were assembled in a one-pot reaction by mixing catenated scaffold and staple strands at final concentrations of 10 nM and 60 nM respectively. Folding buffer contained 5 mM Tris, 1 mM EDTA and 10 mM MgCl₂. To assemble structures, DNA strands were incubated at 80 °C for 10 min followed by cooling from 79 °C to 25 °C with a decrease of 1 °C per 1 min in a thermocycler.

### AFM analysis

1.5 µL of 2 nM of assembled DNA origami sample was applied to freshly cleaved mica and incubated for 1 min followed by addition of 20 µL of folding buffer and immediately by 1.5 µL of 100 mM NiCl₂. The specimen was measured in liquid by AFM (Dimension Icon, Bruker) working in PeakForce QNM^{®} mode. ScanAsyst-Fluid+ probes (Bruker) with nominal spring constant equal to 0.7 N/m and sharpened tip necessary for high-resolution imaging in fluid (nominal radius equal to 2 nm) were used in all the measurements. Figure 3BC were obtained using MutiMode-8 AFM (Bruker) using BL-AC40TS-C2 probe with the same method.

HS-AFM analysis was performed using a bespoke HS-AFM (NanoLSI, Kanazawa University) with a BL-AC10FS-A2 cantilever probe. DNA origami sample was loaded on freshly cleaved and nickel chloride treated mica and imaged in the folding buffer in tapping mode in liquid.

### References

1 G. Gil-Ramirez, D. A. Leigh and A. J. Stephens, Angew Chem Int Ed Engl, 2015. 54, 6110-6150.
2 C. O. Dietrich-Buchecker, J. P. Sauvage and J. P. Kintzinger, Tetrahedron Letters, 1983. 24, 5095-5098.
3 C. O. Dietrich-Buchecker, J. P. Sauvage and J. M. Kern, Journal of the American Chemical Society, 1984. 106, 3043-3045.
4 R. A Bissell, E. Córdova, A. E. Kaifer, J. F. Stoddart, Nature, 1994. 369, 133-137.
5 E. Moulin, L. Faour, C. C. Charmona-Vargas and Nicolas Guiseppone, Adv Mater, 2020. 32, e1906036.
6 K. F. Wagenbauer, F. A. S. Engelhardt, E. Stahl, V. K. Hechtl, P. Stömmer, F. Seebacher, L. Meregalli, P. Ketterer, T. Gerlinger and H. Dietz et al., Chembiochem, 2017. 18, 1873-1885.
7 P. W. K. Rothemund, Nature, 2006. 440, 297-302.
8 S. M. Douglas, I. Bachelet and G. M. Church, Science, 2012. 335, 831-834.
9 M. S. L Tang, S. C. Shiu, M. Godonga, Y. Cheung, S. Liang, R. M Dirkzwager, A. B. Kinghorn, L. A. Fraser, J. G. Heddle and J. A. Tanner, Nanomedicine, 2018.14, 1161-1168.
10S. Brown, J. Majikes, A. Martinez, T. M. Girón, H. Fennell, E. C. Samano and T. H. LaBean, Nanoscale, 2015. 7, 16621-16624.
11 H. Said, V. J. Schüller, F. J. Eber, C. Wege, T. Liedl and C. Richert, Nanoscale, 2013. 5, 284-290.
12A.N. Marchi, I. Saaem, B. N. Vogen, S. Brown and T. H. LaBean, Nano Lett, 2014. 14, 5740-5747.
13Krieg, E. and W. M. Shih, Angew Chem Int Ed Engl, 2018. 57, 714-718.
14 H. Zhang, J. Chao, D. Pan, H. Liu, Q. Huang and C. Fan, Chem Commun (Camb), 2012. 48, 6405-6407.
15M. Erkelenz, D. M Bauer, R. Meyer, C. Gatsogiannis, S. Raunser, B. Saccà and C. M Niemeyer, Small, 2014. 10, 73-77.
16R.M. Zadegan, M. D. E. Jepsen, K. E. Thomsen, A. H. Okholm, D. H. Schaffert, E. S. Andersen, V. Birkedal and Jørgen Kjems, et al., ACS Nano, 2012. 6, 10050-10053.
17F. A. S Engelhardt, F. Praetorius, C. H. Wachauf, G. Brüggenthies, F. Kohler, B. Kick, K. L Kadletz, P. N. Pham, K. L. Behler, T. Gerling and H. Dietz, ACS Nano, 2019. 13, 5015-5027.
18P. M. Nafisi, T. Aksel and S.M. Douglas, Synth Biol (Oxf), 2018. 3, ysy015.
19W. Liu, H. Zhong, R. Wang and N. C. Seeman, Angew Chem Int Ed Engl, 2011. 50, 264-267.
20S. M. Douglas, H. Dietz, T. Liedl, B. Högberg, F. Graf and W. M. Shih, Nature, 2009. 459, 414-418.
21 T. Gerling, K. F. Wagenbauer, A. M. Neuner and H. Dietz, Science, 2015. 347,1446-1452.
22S. Woo, and P. W. K. Rothemund, Nat Chem, 2011. 3, 620-627.
23K. F. Wagenbauer, C. Sigl, and H. Dietz, Nature, 2017. 552, 78-83.
24G. Tikhomirov, P. Petersen, and L. Qian; Nature, 2017. 552, 67-71.
25M. L. Martinez-Robles, G. Witz, P. Hernandez, J. B. Schvartzman, A. Sasiak and D. B. Krimer, Nucleic Acids Res, 2009. 37, 5126-5137.
26J. Elbaz, Z. Wang, F. Wang and I. Willner, Angew Chem Int Ed Engl, 2012. 51. 2349-2353.
27C.H. Lu, A. Cecconello, J. Elbaz, A. Credi and I. Willner, Nano Lett, 2013. 13, 2303-2308.
28D. Liu,G. Chen, U. Akhter, T. M. Cronin and Y. Weizmann, Nat Chem, 2016. 8, 907-914.
29F. Lohmann, , J. Valero and M. Famulok, Chem Commun (Camb), 2014. 50, 6091-6093.
30T.L. Schmidt, and A. Heckel, Nano Lett, 2011. 11, 1739-1742.
31 X. J. Qi, C. H. Lu, X. Liu, S. Shimron, H. H. Yang and I. Willner, Nano Lett, 2013. 13, 4920-4924.
32J. Valero, N. Pal, S. Dhakal, N. G. Walter and M. Famulok, Nat Nanotechnol, 2018. 13, 496-503.
33M. Takaku,D. Takahashi, S. Machida, H. Ueno, H. Hosoya, S. Ikawa, K. Miyagawa, T. Shibata and H. Kurumizaka, Nucleic Acids Res, 2010. 38, 7579-7586.
34 D. Han,S. Pal, Y. Liu and H. Yan, Nat Nanotechnol, 2010. 5, 712-717.
35A. Peil, P. Zhan and N. Liu, Small, 2020. 16, e1905987.
36J. List, E. Falgenhauer, E. Kopperger, G. Paradatscher and F. C. Simmel, Nat Commun, 2016. 7, 12414.
37Q. Xiong, C. Xie, Z. Zhang, L. Liu, J. T. Powell, Q. Shen and C. Lin, Angew Chem Int Ed Engl, 2020. 59, 3956-3960.
38W. M. Stark, D. J. Sherratt and M. R. Boocock, Cell, 1989. 58, 779-790.
39P. H. Arnold, D. G. Blake, N. D. Grindley, M. R. Boocock and W. M Stark, EMBO J, 1999. 18, 1407-14.
40 M. J. Mcllwraith, M. R. Boocock and W. M. Stark, J Mol Biol, 1996. 260, 299-303.
41 F. J. Olorunniji, J. He, S. V. C. T. Wenwieser, M. R. Boocock and W. M. Stark, Nucleic Acids Res, 2008. 36, 7181-7191.
42W. M. Stark,C. N. Parker, S. E. Halford and M. R. Boocock, Nature, 1994. 368, 76-78.
43 N. J. Kilby, M. R. Snaith and J. A. Murray, Trends Genet, 1993. 9, 413-421.
44B. Högberg, T. Liedl and W.M. Shih, J Am Chem Soc, 2009. 131, 9154-9155.
45L. A. Bednarz, M. R. Boocock and D. J. Sherratt, Genes Dev, 1990. 4, 2366-2375
46S. M. Douglas, A. H. Marblestone, S. Teerapittayanon, A. Vazquez, G. M. Church and W. M. Shih et al., Nucleic Acids Research, 2009. 37, 5001-5006.

## Claims

1. A nucleic acid topogami **characterised in that** it is artificial nucleic acid nanostructure consisting of at least 2 interlocked single stranded DNA rings and associated complementary staples strands capable of folding into as-designed 2D or 3D structures.

2. The topogami according to claim 1 where each interlocked ssDNA ring consists of more than 300 nucleotides.

3. The topogami according to claim 1 wherein the interlocked nucleic acid sequences are DNA ("DNA Topogami")

4. The topogami according to claim 1 wherein the interlocked nucleic acid sequences are RNA ("RNA Topogami")

5. The topogami according to claim 1 where the ssDNAs is originating from M13 phage genomic DNA.

6. The topogami according to claim 1 wherein the staple strands are RNA or DNA.

7. The topogami according to claims 1-6 where the folded 2D or 3D structures are DNA or RNA origami structures.

8. The topogami according to claim 1 consisting of 2 interlocked single stranded DNA rings and associated complementary staples, wherein:
- the first interlocked single stranded DNA ring comprises Seq Id. No. 1,
- the second interlocked single stranded DNA ring comprises Seq Id. No. 2,
- the complementary staple associated complementary to first DNA ring comprises sequence selected among of Seq. Id. No. 3-51,
- the complementary staple associated complementary to second DNA ring comprises sequence selected among of Seq. Id. No. 52-122.

9. A method for making interlocked single stranded DNA rings whereby double stranded rings comprising following steps:
a) engineering of DNA plasmid vectors to produce a vector consisting of two scaffold domains connected by parallel recognition sequences (res) in a negatively supercoiled parent plasmid and having integrated on opposite strands of the vector and having two nickase sites, one each on opposite strands of the vector;
b) processing of the plasmid obtained in step a) by Tn3 resolvase, a serine recombinase to catalyse a site-specific recombination at the res sites to form a supercoiled catenane;
c) conversion of catenated product obtained in step b) into ssDNA by Nt.BspQI nicking and subsequent exonuclease digestion, with nicked non-catenated parent plasmid removed by exonuclease digestion;
d) purification of the catenated ssDNA product by phenol:chloroform extraction and concentration by EtOH precipitation.

10. The method according to claim 9, wherein the nickase site used in step a) is the Nt.BspQI nickase site.

11. The method according to claim 9, wherein the exonucleases used in step c) are Exonuclease I and III.

12. The method according to claim 9, wherein the plasmid used in step a) which consists of a plasmid based on pMA21 plasmid which contains two recognition sequences (res) in direct repeat and one NtBspQI nickase site wherein the addition of a second NtBspQI nickase site results in the plasmid named pTopoScaf suitable for processing as describes in 9c.
